Europäisches Patentamt

(19) European Patent Office

Office européen des brevets

(11) Publication number: **0 196 879**

**A2**

# (12) EUROPEAN PATENT APPLICATION

(21) Application number: **86302264.6**

(22) Date of filing: **26.03.86**

(51) Int. Cl.⁴: **C 12 N 15/00**
**//A01K1/00**

(30) Priority: **26.03.85 JP 59640/85**

(43) Date of publication of application:
**08.10.86 Bulletin 86/41**

(84) Designated Contracting States:
**AT BE CH DE FR GB IT LI LU NL SE**

(71) Applicant: **Urata, Shuichi**
**46, Aza Numanosawa Shichinohe-machi**
**Kamikita-gun Aomori-pref.(JP)**

(72) Inventor: **Urata, Shuichi**
**46, Aza Numanosawa Shichinohe-machi**
**Kamikita-gun Aomori-pref.(JP)**

(74) Representative: **Wain, Christopher Paul et al,**
**A.A. THORNTON & CO. Northumberland House 303-306**
**High Holborn**
**London WC1V 7LE(GB)**

(54) **Method of producing hairless pigs.**

(57) Genetic manipulation techniques involving the use of a bacteriophage are employed to alter the genetic structure and form a no-hair gene. Pigs resulting from the union of no-hair gene pigs are hairless (or have just patches of fuzzy hair). The no-hair strain can reproduce itself, and the method can be used to produce hairless strains of the various established breeds. Such pigs have wide utility in many commercial and industrial fields, as well as in research and testing procedures involving substances or properties which are new and are or may be hazardous to life.

EP 0 196 879 A2

Croydon Printing Company Ltd.

- 1 -

## METHOD OF PRODUCING HAIRLESS PIGS

This invention relates to a method for raising (producing) pigs with no body hair or with fuzz.

Conventionally, the reproducible artificial production and raising of pigs having no hair on their skin or only fuzz has been considered impossible. In recent years, however, pig consumption has grown and there has been a dramatic increase in the number of pigs being produced.

This has been accompanied by research into effective raising methods, and the modification of the pig itself has been considered from a number of viewpoints. With regard to body hair in particular, the following problems have been pointed out.

1. Airborne dust and microorganisms stick to the hairs, and the motion of of the pig causes such dust and microorganisms to float up in the air and be inhaled, thereby becoming the cause of various illnesses.

2. It is easy for ticks, lice, mites and other such external parasites to live on the hair, and they may also enter the body.

3. Disinfection is an effective way of protecting pigs from disease, but it is difficult to disinfect the skin and down to the hair roots.

4. When it comes to dealing with excrement and at processing time, the removal of the pig-hair is a difficult and time-consuming task.

5. When the pigs are slaughtered and cut up, it is impossible to remove all the hairs from where the edible portions have been cut, and hairs may be overlooked, leading to subsequent problems. The value of such meat for sale or serving is reduced, and can cause revulsion, loss of appetite, complaints and claims for compensation.

6. Skin with the hairs in it is difficult to process into food.

7. Ears and tails with hairs are difficult to process into food.

8. Because there is also hair on the pig's heads, head meat processing yield is low and takes time, and is therefore inefficient.

9. The skin of a hairy pig has many hair roots, and because these roots are difficult to remove completely, good-quality gelatin, a source of protein, cannot be extracted therefrom.

**0196879**

10. When pigs with hair are used to obtain specific pathogen-free or sterile pigs, when the fetus is removed using ceasarean section, hysterotomy, hysterectomy, abdominal section and other such surgical procedures, it is necessary to shave around the area of incision.

11. When pigs with hair are used as the subjects of skin-related experiments, research and instruction, the hair is a hindrance.

12. Because pigs with hair have many antibodies, they connot be readily used for medical experiments.

13. When pigs suffer burns and other injuries to the skin, the area has to be shaved before it can be treated.

14. Pigs with hair connot be readily used in experiments relating to industrial science and radioactivity.

15. In the case of both pigs with and without hair, there is no technology for raising the pigs to be large, medium-sized or small, in accordance with a specific purpose.

16. Ordinarily, pigs are not kept as pets.

The feasibility of producing hairless pigs or pigs with fuzzy hair around the legs and tail to eliminate the above problems has been examined, but owing to problems involving achieving consistent reproducibility, no concrete method of producing hairless pig was perfected.

The object of the present invention is to provide a method for producing hairless pigs that overcomes the above drawbacks 1 to 16 of the conventional art. Pigs with heritable qualities such as no hair or sparse, short fine, soft hair, rapid growth, meaty except around head and feet, and have red meat in fatty portions and marbled red meat, and the sows of which are very fertile, having many teats and producing many youg, can be utilized in a number of industrial areas, including pig-raising, food processing, chemical processing, medicine, pharmacology, and in experimental research involving radioactivity and nuclear power. Such pigs can also be utilized in experiments, research and tests involving nuclear power and related radioactivity, drugs, medical products, new organisms produced by gene conversion and other such areas involving direct or indirect risk to the life of animal and other organisms.

In particular, it is known that pig skin is very like human skin, and such pigs will therefore have wide applicability as animals for use in experiments, research and tests.

According to one aspect of the present invention there is provided a method of producing hairless pigs comprising the steps of removing one or more fertilised ova from a sow, introducing a bacteriophage into the DNA of the hair producing chromosome in the fertilised nucleus whereby the DNA becomes a non-hair gene or inherited gene thereof, and replacing the treated ova into the or another sow.

According to a second aspect of the present invention there is provided a method of producing hairless pigs obtained by mating pigs possessing no-hair genes with other pigs possessing no-hair genes.

There are two types of no-hair gene pigs. One type has a latent no-hair gene, which may not be manifested externally, so the pig may have hair; the other type actually displays a hairless appearance. A possible method of producing a pig that has hair but which possesses the no-hair gene, is to take a hairless pig produced by the method of the present invention, said pig possessing the no-hair gene, and cross it with an ordinary hairy pig. Basically, however, in a hairy pig which has been provided with the no-hair gene of a hairless pig by genetic manipulation, the no-hair gene is latent. That is, incorporating the genes of a hairless pig into the genes of a hairy pig produces a hairy pig which has no-hair genes. Mating or artificial fertilization is used to make the pig pregnant. The fertilized egg is extracted from the uterus and manipulation of the genes, etc., is carried out, or the ovum is removed from the uterus and fertilization and genetic and sex manipulation performed externally and the fertilized egg is implanted into a sow used exclusively for breeding to mass-produce pigs of the same sex and quality in a short time.

The method of manipulating the genes of a hairless pig into the genes of a hairy pig comprises the following.

When the breeding takes place, the nucleus of the fertilized egg contains the various chromosomes and DNA material of the genes. Among these chromosomes is one that results in the pig having hair. A bacteriophage (a virus that infects and kills only bacilli; in appearance, it consists of a hexagonal adductor and insect-like hairs, and uses its tail to break the cell wall, enter and multiply) is introduced into the DNA of that chromosome, whereupon the bacteriophage multiplies, breaking down part of the chromosome's DNA, the bacteriophage thereby acting as a restriction enzyme (an enzyme which cuts the DNA chain at a specific base sequence). The result of breaking the sequence of the hair gene is to covnert it into a no-hair gene.

Shown in Table 1-1 are hypothetical genetic manipulations for the addition and replication of DNA segments, and Table 1-2 shows estimated junctions of no-hair DNA sections.

0196879

Table 1 — 1

Manipulation of estimated gene (addition and replication
of estimated DNA segment)

(1) DNA sequence of ordinary gene

DNA + [TTTTTTTTTTTTTTTT]

C C A G T G A A T T A C A A A

Reverse transcriptase ↑

G G U C A C U U A A U G U U U

mRNA − [IIIIIIIIIIIIIIII]

(2) Hair estimated gene

G ｜A  A  T  T  C        G ｜A  A  T  T  C

C  T  T  A  A ｜G        C  T  T  A  A ｜G

(3)'Cutting by restrictor enzyme (hair estimated DNA)

｜A  A  T  T  C                    G ｜

｜G                          C  T  T  A  A ｜

Table 1 — 2

0196879

Junction of small no-hair DNA segment

```
                    A   A   T   T   C
Plasmid (Vector)   ┌───────────────────┐
(Loop DNA)         │   │   │   │   │   │
                   │   │   │   │   │   └────┐
                                           └────
```

Small no-hair DNA segment

```
 A   A   T   T   C               G
┌───┬───┬───┬───┬───────────────┬───┐
│   │   │   │   │               │   │
│   │   │   │   │               │   │
└───────────────┘               │   │ │ │ │ │
                                └───┴─┴─┴─┴─┴──
                                  C   T   T   A   A
```

Plasmid (Vector)
(Loop DNA)

```
┌───────────────────┐
│                   │
│                   │ │ │ │ │
└───────────────────┴─┴─┴─┴─┴──
                      C   T   T   A   A
```

0196879

- 7 -

In performing the genetic manipulation, prior to the mating calcium chloride, a colorless calcium salt that readily dissolves in water, is administered orally, in combination with a 0.1M calcium chloride culture solution of coli into the uterus. Preferably, also, following mating, the body temperature is rapidly raised to around 37°C by fighting or the like, then abruptly cooled, so that the walls (cells) of the coli in the uterus are suitably broken down, and dissolved by the bacteriophage, facilitating parasitism.

The bacteriophage enters by dissolving part of the chromosomal genes (plasmids, which are loops of DNA in the coli cells). As a result, part of the DNA of the hair gene is dissolved, giving rise to no-hair chromosomal genes. That is, a no-hair chromosomal gene is produced in the DNA loop which is cut by bacteriophagic action.

The preferred bacteriophage is a corona virus of TGE (T. Epdic Gastroenteristis) parasitic on Coli and a reovirus of weak toxin rotta on Coli.

The preferred method uses fertilised ova, which may be fertilised by mating or by artificial insemination. Within 24 to 32 hours after fertilisation, one or more fertilised ova are removed. The sow's uterus is disinfected and irrigated, then the disinfectant is washed out with buffer solution. The buffer solution is injected into the uterus by means of a balloon catheter and the outflow is collected. From the collected outflow one or more ferti ova can be selected and removed for treatment with the bacteriophage.

The fertilised ova is dissolved or placed in calcium chloride solution and the existing plasmids confirmed. After cooling, the bacteriophage is added and attacks the ovum by tracing a portion of a spermatozoon. The treated ova can then be replaced into the same or another sow. The bacteriophage affects preferentially the 61-83 aminoacids which are those which control hair growth.

For external fertilisation, one or more ova (unfertilised) are removed from a sow during ovulation after esters. Spermatozoa removed from a boar and kept without buffer solution are added to the ova. During fertilisation the spermatozoa dissolve a part of the ovum membrane to enter within, the bacteriophage entering through the same dissolved portion. The treated, fertilised ova are then implanted.

Even when a hair gene is dissolved and a no-hair gene produced, there are 1.5 to 2 billion spermatozoa in the semen of one ejaculation, which in the case of two matings therefore rises to 3 to 4 billion, among which many differences, both great and small, can be found (differences between individual pigs and between semens). With 3 or 4 billion spermatozoa to fertilize several dozen ova, the probability involved is about 1% of one in 3 or 4 billion. However, the formation of no-hair genes can be achieved by reproducible artificial methods.

As shown in Table 1-1, by the dissolving of the hair gene and the reverse transcriptase from the messenger RNA (back-synthesis from mRNA of DNA bearing the genetic code) no-hair genetic plasmid DNA (gene vector, one-hundredth of the chromosomal DNA) and viral DNA are incorporated into the mRNA. The no-hair gene is transferred, through bacterial contact, to another bacillus which, now with the no-hair gene, enters the chromosomes of hair genes, and therein multiplies. This gives rise to the basic genes having hair genetic molecules and no-hair genetic molecules. Contained in each fertilized cell nucleus is the genetic information that is essential for such vital phenomena as multiplication, differentiation and metabolism. Also, the varied structures of the fertilized nucleus possessing hair and no-hair genetic molecules are believed to be related to the appearance of hairless pigs. The possibility of differentiation of no-hair genes signifies a stability of their structure and function, and the physiological foundation of the no-hair genes lies in the synthesis of special proteins.   Regarding the making of specific proteins, because of the communicative stimulus from the special no-hair gene, and controlling stimulus from proteins in the organism such as enzymes, antibodies, receptors, and repressors, the protein molecules can exist in a higher form, and as the hairless appearance can be changed by external factors, the hairless pig responds to the stimulus of the outside world, and antibodies can provide protection with respect to external changes.   Proteins formed thus have various effects and morphologies.   The differentiation into different organisms of nucleic cell molecules of fertilized eggs possessing hair genes and no-hair genes is attested to by the actual birth of hairless pig with a different genetic structure, and with changes in genetic character being an evolution from   the origin of the pig species   , the nature of the hairless pig is decided by genetic DNA molecules.   That is, a hairless pig has altered genetic DNA molecules.   This is a matter of course, and is not a chance mutation.

As animal examples, there are the nucleus transfer experiments of J. B. Gurden, involving deactivating cells of unfertilized eggs of the African frog (Xenopus laevis) by ultraviolet radiation, and implanting in these cells cytoplasm-free nuclei taken from the outer wall of the small intestine of tadpoles.   Some of the egg cells with the transplanted nuclei underwent normal development, surviving to become frogs.   The reason for selecting cells of the intestinal outer wall is that they have stripes enabling it to be confirmed that the cells have undergone full differentiation. A large proportion of egg cells with nuclei transplanted from cells in the initial development period developed normally.   In the experiments in which the transplanted nuclei were taken from cells in which differentiation had progressed, there were many cases of arrested development or deformity.   This is owing to the fact that the more specialized the cell, the more difficult it is to effectively remove and transplant the nucleus.

Tadpoles which developed from transplanted nuclei did not exhibit the genetic characteristics of the cytoplasm of the cell from which the nucleus came. This fact was further demonstrated by taking cell nuclei from colored tadpoles and implanting them into the eggs of non-colored tadpoles, resulting in colored offspring only.   On the basis of these transplantation studies it has been postulated that genes are present within the cell nucleus, bu that their manifestation is regulated by the cytoplasm.    It became clear that by transplanting the nucleus of a brain cell or mature erythrocyte which normally did not synthesize DNA to an egg from which the nucleus had been removed resulted in DNA synthesis.    From this it became clear that all the genes in a fertilized egg are maintained in their full form throughout the life of that organism, and that the manifestation thereof is decided by the properties of the cytoplasm.  (P. 99  Seimei Kagaku, ed. Prof. Yamafuji)

That is, if the results of each of two matings between the same sow and male pig are hairless pigs of the same character and type, this demonstrates the correct order of the genetic process.    This is one-way differentiation of no-hair genes, promoted by artificial means.    That is, it is the orderly, unidirectional genetic process in pigs possessing no-hair genes, enabling the artificial production of hairless pigs by the mating of pigs each of which possess no-hair genes.

Because the no-hair gene is recessive, it does not show externally that it has been inherited.    This manifestation is done by the following method.

A pig's development, physiology, presence or absence of hair and other quantitative features are a result of heredity and environment.  While the character is controlled by the genes, the expression thereof is affected by the body of the mother, the raising process, the controlling environment, atmospheric pressure, climate and the like; phenotype selection is not solely a genetic function.

Regarding pigs that can be inferred to possess the no-hair gene, without recourse to Mendelian laws, the F1 product will be a product of the parents, and it can be inferred that it is related to many gene multiples of the no-hair genetic character.

With regard to the numerical characters of the hairless pig, with the overall large numbers of genes involved, the effect of the character expression thereof is such that the effect of dominance, recessiveness, homeostasis, heterostasis, etc. of large numbers of genes does not pose much of a problem.

It is postulated that in hairless pigs having no-hair genes, the expression thereof was by polygenes.  The following mating method was used to implement this.

By combining variant mutagens produced by continuous interbreeding with variant mutagens produced by continuous regressive interbreeding, the mating variant induction effect is synergized by the no-hair mutagenic effect of each side, causing expression of the no-hair character. That is, the hair gene being dominant, in the case of one gene, it is the character of the hair gene that is expressed. On the other hand, the no-hair gene being regressive, the synergistic effect alters the hair gene so that the recessive no-hair gene changes to the phenotype, and is expressed. That is, with an expressed no-hair recessive gene and a dominant hair gene, the character of the dominant hair gene is expressed. In the case of a pair having the no-hair recessive gene and the dominant hair gene, the result is the following.

1)    In the case of no-hair recessive gene and no-hair recessive gene, expression is of the no-hair character.

2)    In the case of no-hair recessive gene and dominant hair gene, expression is of the hair character.

3)    In the case of dominant hair gene and dominant hair gene, expression is of the hair character.

From this it follows that in the case of pigs of the same species, mating a hairless type with a hairy type imposes the genetic character of the hairless type on the qualities of the hairy type, providing a hairless pig type that has no-hair genes, and mating this type with another identical type which has the no-hair genes produces a hairless pig.

In the drawings:

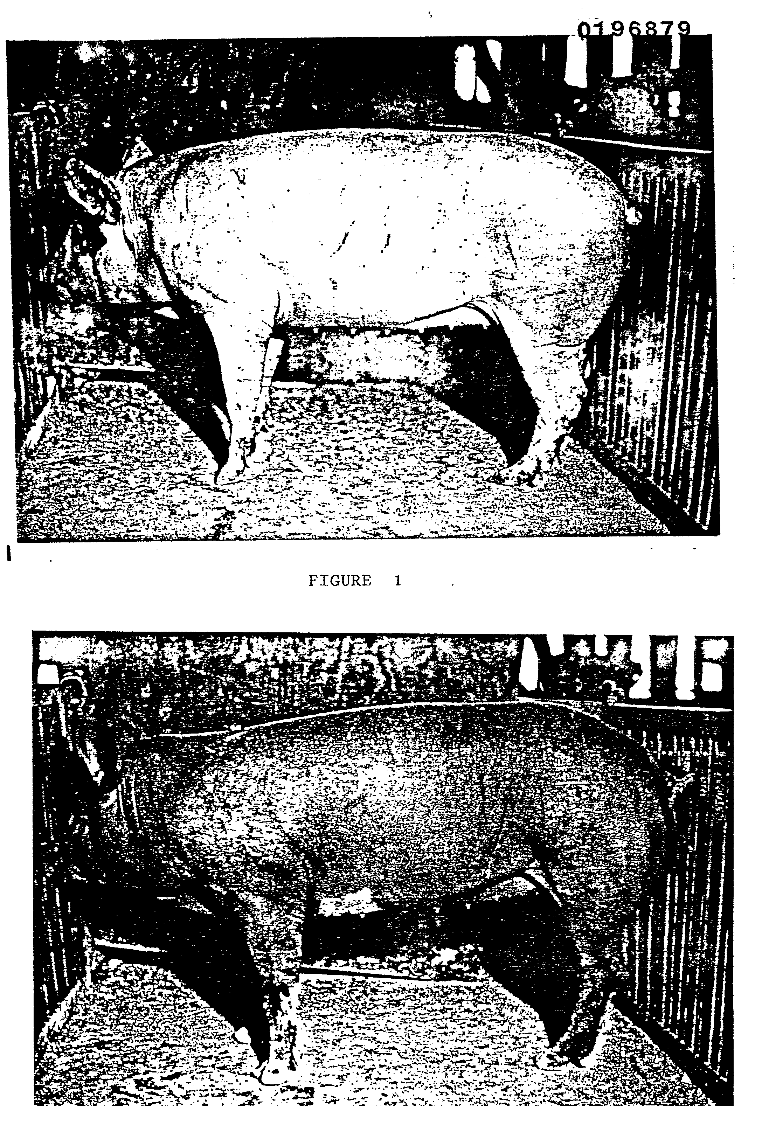

Fig. 1 is a photograph of a living hairless pig (female 228-11) produced in accordance with the present invention; and Fig. 2 is a photograph of another hairless pig (female 228-12).

Research started in 1973 and resulted in birth of hairless pigs in 1983. In August 1984, a sow with no-hair hereditary capability which had given birth to 4 hairless offspring was mated with a male with no-hair hereditary capability, resulting, on January 1, 1985, in the birth of 3 hairless young, proving beyond doubt the fecundity and inheritability of the pigs produced thus.

Preferred embodiments of the invention will now be more particulargly described.

1. Table 1-3 lists the matings of Hampshires and Landraces each possessing no-hair genes.

Table 1 — 3

| AA : H strain | Hampshire | Hair Gene |
|---|---|---|
| a a : H strain | Hampshire | No-hair Gene |
| CC : L strain | Landrace | Hair Gene |
| c c : L strain | Landrace | No-hair Gene |

AA a a × CC c c
Gametes A a     C c
F 1       A a C c

F 2

| Gametes | A C | A c | a C | a c |
|---|---|---|---|---|
| A C | AACC | AACc | AaCC | AACc |
| A c | AACc | AAcc | AaCc | Aacc |
| a C | AaCC | AaCc | aaCC | aaCc |
| a c | AaCc | Aacc | aaCc | aacc |

| Gene No. | Estimated Gene | Number of Phenotypes | Phenotype Breed | Estimated Gene Value | Hair present/ Absent | Real Number of Phenotypes |
|---|---|---|---|---|---|---|
| 1 | A A C C | 1 | H L | H L | present | 1 |
| 2 | A A C c | 2 | H L | H L | present | 2 |
| 3 | A A c c | 1 | H L | H L | present | 1 |
| 4 | A a C C | 2 | H L | H L | present | 2 |
| 5 | A a C c | 4 | H L | H L | present | 3 |
| 6 | A a c c | 2 | H | H L | present | 2 |
| 7 | a a C C | 1 | L | H L | present | 1 |
| 8 | a a C c | 2 | L | H L | present | 1 |
| 9 | a a c c | 1 | H L | H L | Absent | 0 |
| | | 1 6 | | | | 1 3 |

All F1 offspring between hairless and hairy types were hairy, but F2 produced both hairless and hairy pigs. It is because the no-hair gene is regressive and the hair gene dominant that F1 produced only hairy pigs. However, pigs with no-hair genes were reared.

2. Because of the recessiveness no hairless pigs appeared in the first generation (F1) of hybrids, but in the second generation (F2) such pigs accounted for just one-quarter of the total.

3. Hybridization, mating of first generation (F1) hairless pigs gave rise in the second generation to a ratio of three hairless pigs to one hairy pig.

4. Backcrossing of a hairy pig (F1) which has the recessive no-hair gene with a parent which has the recessive no-hair gene gave rise to a hairless-hairy ratio of 1 : 1.

5. The recessive hairless gene is independent.

6. Table 1-3 example:

Mating no-hair-gene Hampshire male (F1) with a (Landrace male x Hampshire female) sow produced a hairless Hampshire line, a Landrace x Hampshire (F1) line, a hairy Hampshire line, and a Landrace x Hampshire (F1) line. By body type, also, there were sepatate, independent no-hair and hair genes for large, medium-size and small types.

7. If genetic symbols are used to describe the mating principles, then:

AA: Hair gene

aa: No-hair gene

BB: Robust gene

bb: Weak gene

Hairy pig　　x　　Hairless pig AABB　　x　　aabb Gametes  AB　　　　ab F1　　　　　AaBb

F2  Gametes

|    | AB   | Ab   | aB   | ab   |
|----|------|------|------|------|
| AB | AABB | AABb | AaBB | AaBb |
| Ab | AABb | AAbb | AaBb | Aabb |
| aB | AaBB | AaBb | aaBB | aaBb |
| ab | AaBb | Aabb | aaBb | aabb |

| GENOTYPE | | PHENOTYPE | FIRST LITTER | SECOND LITTER |
|---|---|---|---|---|
| AABB | 1 | Hairy/Robust | 1 | 1 |
| AABb | 2 | Hairy/Middling strength | 2 | 1 |
| AAbb | 1 | Hairy/Weak | 1 | 1 |
| AaBB | 2 | Hairy/Robust | 2 | 2 |
| AaBb | 4 | Hairy/Middling strength | 3 | 2 |
| Aabb | 2 | Hairy/Weak | 1 | 2 |
| aaBB | 1 | Hairless/Robust | 1 | 1 |
| aaBb | 2 | Hairless/Middling strength | 2 | 2 |
| aabb | 1 | Hairless/Week | 1 | 2 |
| | 16 head | | 14 head | 13 head |

(Dam No. 61)                    (Boar No. 2-5)

First litter born August 1984; scond litter born January 1985

—A brief explanation will now be given of the mating charts and the effect of the present invention on phenotypes. —

The effectivre action of the no-hair genes is proportional to their numbers, and does not vary even if they are substituted. With respect to Table 1-3, it is estimated that the gametes Aa and Cc have a single breed effect, and that AC, Ac, aC and ac have a two-breed effect.

Table 2 shows the mating of Hampshires (Aa) and large Whites (Bb). The estimated effect of gametes Aa and Bb is 1 breed with respect to F1, AB, Ab, aB and ab show a 2-breed effect.

Regarding mating of Hampshires (Aa) and Hampshires (Aa), with gametes Aa and Aa, F1 is AAaa, showing a 1-breed effect, and appears as H = Hampshire (AAaa) = Aa.

In Table 1-3, with regard to the mating of Hampshires (Aa) and Landraces (Cc), gametes Aa and Cc are AaCc at F1, showing a 2-breed effect. Hampshire phenotype effects in evidence include the ears, while around the eyes and on the rear of the body are black patches and the body is longer than that of a Hampshire and shorter than that of a Landrace.

In Table 2, regarding the effect of Hampshires (Aa) and large White (Bb), gametes Aa and Bb are AaBb at F1, the effect being to show the characteristics of both breeds.

Table 2

| AA : | Hampshire | Hair Gene |
|------|-----------|-----------|
| a a : | Hampshire | No-hair Gene |
| BB : | Large White | Hair Gene |
| b b : | Large White | No-hair Gene |

AAaa  ×  BBbb

Gametes   A a          B b

F 1              AaBb

F 2

| Gametes | A B | A b | a B | a b |
|---------|-----|-----|-----|-----|
| AB | AABB | AABb | AaBB | AABb |
| Ab | AABb | AAbb | AaBb | Aabb |
| aB | AaBB | AaBb | aaBB | aaBb |
| ab | AaBb | Aabb | aaBb | aabb |

| Gene No. | Estimated Gene | Number of Phenotypes | Phenotype Breed | Estimated Gene Value | Hair Present/ Absent | Real Number of Phenotypes |
|----------|----------------|----------------------|-----------------|----------------------|----------------------|---------------------------|
| 1 0 | AABB | 1 | WH | WH | Present | 1 |
| 1 1 | AABb | 2 | WH | WH | Present | 1 |
| 1 2 | AAbb | 1 | H | WH | Present | 1 |
| 1 3 | AaBB | 2 | WH | WH | Present | 2 |
| 1 4 | AaBb | 4 | WH | WH | Present | 3 |
| 1 5 | Aabb | 2 | H | WH | Present | 2 |
| 1 6 | aaBB | 1 | W | WH | Present | 1 |
| 1 7 | aaBb | 2 | W | WH | Present | 1 |
| 1 8 | aabb | 1 | WH | WH | Absent | 0 |
| | | 1 6 | | | | 2 |

Regarding phenotype values, the shape of the face is Hampshire, overall the coloring is large White, and the body type is a mixture of Hampshire and large White, being half and half, and around the eyes and on the rear part are black patches. This thus shows the phenotypical effects of both breeds. The arithmetic effect is such that substituting males and females does not change the effect very much.

Table 3 shows the following effects of three types of estimated gene values from the mating of AaBb (HW) and AaCc (HL) having the estimated genes.

| AABC | AABc | AAbC |
|------|------|------|
| AAbc | AaBC | AaBc |
| AabC | Aabc | aaBC |
| aaBc | aabC | aabc |

Substitution produces a difference in the effect of the estimated gene value. That is because the dominance or recessiveness of the alleles causes the manifestation of hair in the case of the dominant effect of the dominant genes of the particular breed, or the manifestation of no hair in the case of the recessive effect of the recessive genes of the particular breed.

## Table 3

| Gene No. | 5 | Gene No. | 1 4 |
|---|---|---|---|
| Phenotype Breed | H L Strain | Phenotype Breed | HW Strain |
| Estimated Gene | A a C c | Estimated Gene | A a B b |
| Gametes | A C, A c, a C, a c | AB, A b, a B, a b | |

| | AB | A b | a B | a b |
|---|---|---|---|---|
| A C | AABC | AAbC | AaBC | AabC |
| A c | AABc | AAbc | AaBc | Aabc |
| a C | AaBC | AabC | aaBC | aabC |
| a C | AaBc | Aabc | aaBc | aabc |

| Gene No. | Estimated Gene | Number of Phenotypes | Phenotype Breed | Estimated Gene Value | Hair Present/ Absent | Real Number of Phenotypes |
|---|---|---|---|---|---|---|
| 1 9 | AABC | 1 | HWL | HWL | Present | 1 |
| 2 0 | AABc | 1 | HW | HWL | Present | 1 |
| 2 1 | AAbC | 1 | HL | HLW | Present | 1 |
| 2 2 | AAbc | 1 | H | HWL | Present | 1 |
| 2 3 | AaBC | 2 | HWL | HWL | Present | 1 |
| 2 4 | AaBc | 2 | HW | HWL | Present | 1 |
| 2 5 | AabC | 2 | HL | HLW | Present | 1 |
| 2 6 | Aabc | 2 | H | HWL | Present | 2 |
| 2 7 | aaBC | 1 | WL | WLH | Present | 1 |
| 2 8 | aaBc | 1 | W | WHL | Present | 1 |
| 2 9 | aabC | 1 | L | LHW | Present | 1 |
| 3 0 | aabc | 1 | HWL | HWL | Absent | 1 |
| | | 1 6 | | | | 1 3 |

0196879

To continuously produce hairless pigs, with reference to Table 4, the gene No. 26 Aabc males of Table 3 (phenotypes were hairy H strain, estimated genes were H, W, L) were mated with AaBb dams (phenotypes were Fl HW strain, estimated genes were H, W). The result, as shown in the table, was the appearance in a ratio of 6 : 1 of hairless pigs with the estimated recessive genes (aabb, aabc).

0196879

T a b l e  4

| Gene No. | 2 6 | Gene No. | 1 4 |
|---|---|---|---|
| Phenotype Breed H (♂ 2 − 5) | | Phenotype Breed    W H | |
| Estimated Gene    A a b c | | Estimated Gene    A a B b | |
| Gametes | A b, A c, a b, a c | AB, Ab, aB, ab | |

| | AB | Ab | aB | ab |
|---|---|---|---|---|
| A b | A A B b | A A b b | A a B b | A a b b |
| A c | A A B c | A A b c | A a B c | A a b c |
| a b | A a B b | A a b b | a a B b | a a b b |
| a c | A a B c | A a b c | a a B c | a a b c |

| Gene No. | Estimated Gene | Number of Phenotypes | Phenotype Breed | Estimated Gene Value | Hair Present/ Absent | Real Number of Phenotypes |
|---|---|---|---|---|---|---|
| 3 2 | A A B b | 1 | HW | HW | Present | 1 |
| 3 3 | A A B c | 1 | HW | HWL | Present | 1 |
| 3 4 | A A b b | 1 | H | HW | Present | 1 |
| 3 5 | A A b c | 1 | H | HWL | Present | 1 |
| 3 6 | A a B b | 2 | HW | HW | Present | 1 |
| 3 7 | A a B c | 2 | HW | HWL | Present | 2 |
| 3 8 | A a b b | 2 | H | HW | Present | 1 |
| 3 9 | A a b c | 2 | H | HWL | Present | 2 |
| 4 0 | a a B b | 1 | W | WH | Present | 1 |
| 4 1 | a a B c | 1 | W | WHL | Present | 1 |
| 4 2 | a a b b | 1 | HW | HW | Absent | 1 |
| 4 3 | a a b c | 1 | HWL | HWL | Absent | 1 |
| | | 1 6 | | | | 1 4 |

* Dam No. 34 ( Gene No. 34 )    * Dam No. 61 ( Gene No. 41 )

Table 5 shows an example of the mating of the gene No. 26 Aabc males (phenotypes were hairy H strain, estimated genes were H, W, L) with gene number 37 AaBb dams (phenotypes were HW strain, estimated genes were H, W, L). In this case, the result was the appearance in a ratio of 6 : 1 of hairless pigs with the estimated recessive genes aabc (H strain, estimated genes H, L) and aacc (HL strain, estimated genes H, L).

Table 5

| Gene No. | 3 9 | Gene No. | 3 7 |
|---|---|---|---|
| Phenotype Breed | H strain | Phenotype Breed | HW strain |
| Estimated Gene | A a b c | Estimated Gene | A a B c |
| Gametes | A b, A c, a b, a c | AB, A c, a B, a c | |

| | A B | A c | a B | a c |
|---|---|---|---|---|
| A b | A A B b | A A b c | A a B b | A a b c |
| A c | A A B c | A A c c | A a B c | A a c c |
| a b | A a B b | A a b c | a a B b | a a b c |
| a c | A a B c | A a c c | a a B c | a a c c |

| Gene No. | Estimated Gene | Number of Phenotypes | Phenotype Breed | Estimated Gene Value | Hair Present/ Absent | Real Number of Phenotypes |
|---|---|---|---|---|---|---|
| 4 4 | A A B b | 1 | HW | HW | Present | 1 |
| 4 5 | A A B c | 1 | HW | HWL | Present | 1 |
| 4 6 | A A b c | 1 | H | HWL | Present | 1 |
| 4 7 | A A c c | 1 | H | HL | Present | 1 |
| 4 8 | A a B b | 2 | HW | HW | Present | 1 |
| 4 9 | A a B c | 2 | HW | HWL | Present | 1 |
| 5 0 | A a b c | 2 | H | HWL | Present | 2 |
| 5 1 | A a c c | 2 | H | HL | Present | 2 |
| 5 2 | a a B b | 1 | W | WH | Present | 1 |
| 5 3 | a a B c | 1 | W | WHL | Present | 1 |
| 5 3 | a a b c | 1 | HWL | HWL | Absent | 1 |
| 5 5 | a a c c | 1 | HL | HL | Absent | 1 |
| | | 1 6 | | | | 1 4 |

In Table 6, two matings between the same boar and dam resulted each time in progeny having the same appearance and body type. This is explained below with reference to the table.

Mating Aabc (phenotype strain, estimated gene values H, W, L) with aaBc (phenotype W strain, estimated gene values H, W, L) produces Ab, Ac, ab, ac aB, and ac gametes. The first litter consisted of 10 pigs with hair and 4 without; the second litter consisted of 9 pigs with hair and 4 without.

The reference photograph affixed to the specification as Fig. 1 is that of a living hairless pig (female 228-11) born in accordance with the present embodiment. Reference photograph as Fig. 2 is also that of a hairless pig (female 228-12) born at the same time.

The hairless pigs in these photographs were born in August 1984 as a result of the first breeding. Judged on the basis of these results, these hairless pigs appeared in accordance with the estimated genes.

Table 6

0196879

| Gene No. | 26 (♂2-5) | Gene No. | 41 (♀61) |
|---|---|---|---|
| Phenotype Breed | H strain | Phenotype Breed | W strain |
| Estimated Gene | A a b c | Estimated Gene | a a B c |
| Gametes | A b, A c, a b, a c | AB, Ac, aB, ac | |

|  | A b | A c | a b | a c |
|---|---|---|---|---|
| a b | A a B b | A a B c | a a B b | a a B c |
| a c | A a b c | A a c c | a a b c | a a c c |
| a B | A a B b | A a B c | a a B b | a a B c |
| a c | A a b c | A a c c | a a b c | a a c c |

| Gene No. | Estimated Gene | Number of Phenotypes | Phenotype Breed | Estimated Gene Value | Hair Present/ Absent | Real Number of Phenotypes A — B | |
|---|---|---|---|---|---|---|---|
| 5 6 | A a B b | 2 | HW | HW | Present | 2 | 1 |
| 5 7 | A a B c | 2 | HW | HWL | Present | 1 | 1 |
| 5 8 | A a b c | 2 | H | HWL | Present | 2 | 2 |
| 5 9 | A a c c | 2 | H | H L | Present | 2 | 2 |
| 6 0 | a a B b | 2 | W | WH | Present | 2 | 2 |
| 6 1 | a a B c | 2 | W | WHL | Present | 1 | 1 |
| 6 2 | a a b c | 2 | W | HWL | Absent | 2 | 2 |
| 6 3 | a a c c | 2 | HL | H L | Absent | 2 | 2 |
| | | 1 6 | | | | 1 4 | 1 3 |

Estimated No. of Pigs with No-hair Expression (12: 4)
Actual No. of Pigs with No-hair Expression (10: 4)
(9: 4) 1984. 8. 1    A
1985. 1. 1    B

With reference to Table 7, mating hairless pig aabc with the estimated recessive gene gene number 31 with hairless pig aabc number 43 to produce further hairless pigs, the result was the following, all of which were hairless.

| | | | |
|-----|---|----------------------------|-----------|
| aabb | 4 | estimated phenotype value | HW strain |
| aabc | 8 | estimated phenotype value | H strain  |
| aacc | 4 | estimated phenotype value | HL strain |

In Tables 1 to 7, F1 gave rise to hairy pigs, the dominant type, in accordance with the laws governing dominant/recessive types. In accordance with the rules of divergency, F2 produced hairy and hairless pigs.

In accordance with the rules of independence, with respect to the estimated genes of hairless pigs, the following can be given:

For hairless pigs,

H strain (aabc)

HL F1 strain (aacc)

HW, F1 strain (aabb)

For hairy pigs,

H strain (AABC, AABc, AAbC, AAbc, Aabc)

HL F1 strain (Aabc)

HW, F1 strain (AaBC, AaBc)

LW, F1 strain (aaBC)

L strain (aabc)

W strain (aaBc)

Accordingly, this shows the independence of phenotypical hair and no-hair genes.

Table 7

| Gene No. | 3 1 | Gene No. | 4 3 |
|---|---|---|---|
| Phenotype Breed | H W L | Phenotype Breed | H W L |
| Estimated Gene | a a b c | Estimated Gene | a a b c |
| Hair Present/Absent | Absent | Hair Present/Absent | Absent |
| Gametes | a b, a c | | a b, a c |

|     | a b | a c | a b | a c |
|-----|-----|-----|-----|-----|
| a b | a a b b | a a b c | a a b b | a a b c |
| a c | a a b c | a a c c | a a b c | a a c c |
| a b | a a b b | a a b c | a a b b | a a b c |
| a c | a a b c | a a c c | a a b c | a a c c |

| Gene No. | Estimated Gene | Number of Phenotypes | Phenotype Breed | Real Number of Phenotypes | Hair Present/ Absent |
|---|---|---|---|---|---|
| 6 4 | a a b b | 4 | H W | H W | Absent |
| 6 5 | a a b c | 8 | H | H W L | Absent |
| 6 6 | a a c c | 4 | H L | H L | Absent |
| | | 1 6 | | | |

—Providing the no-hair genes of one breed of pig to another strain to produce hairless pigs of the other strain —

Tabel 8 shows a method of taking the estimated no-hair genes of one type of pig to provide another type of pig with the no-hair genes.    Estimated phenotype aabc is H strain, estimated genes are H, W, and L.    The estimated phenotypes of the receptor pig were D strain, and estimated genes were D, d.

aabc gametes are ab and ac; DDdd gametes are Dd; F1 are abDd and acDd.    The gametes are aD, ad, bD, bd, aD, ab, cD, and cd.    In accordance with these, estimated no-hair genes are implanted.    Regarding the D strain, all estimated phenotype values were expressed.    The D strain had a 3 : 1 estimated gametic value ratio, 12 hairy and 4 hairless pigs.

The result of repeated continuing experiments was that immediately prior to the filing of the application was that 1 hairless Hampshire and 1 hairless Large White were among 5 Hampshires and 4 Large Whites born to the gene number 81-1 dam and gene number 2-5 pig.

## Table 8
### Producing a hairless breed of pig by applying no-hair genes to a hairy breed.

| Gene No. | 6 5 | Gene No. | 0 |
|---|---|---|---|
| Phenotype Breed | H | Phenotype Breed | D (Estimated Breed) |
| Estimated Gene | a a b c | Estimated Gene | D D d d |
| Hair Present/Absent | Absent | Hair Present/Absent | Present |
| Gametes | a b, a c | | D d |
| F 1 | a b D d, a c D d | | |
| Gametes | a D, a d, b D, b d | a D, a d, c D, c d | |

| | a D | a d | c D | c d |
|---|---|---|---|---|
| a D | a a D D | a a D d | a c D D | a c D d |
| a d | a a D d | a a d d | a c D d | a c d d |
| b D | a b D D | a b D d | b c D D | b c D d |
| c d | a c D d | a c d d | c c D d | c c d d |

| Gene No. | Estimated Gene | Number of Phenotypes | Phenotype Breed | Estimated Gene Value | Hair Present/Absent |
|---|---|---|---|---|---|
| 6 7 | a a D D | 1 | D | D A | Present |
| 6 8 | a a D d | 2 | D | D A | Present |
| 6 9 | a b D D | 1 | D | D H W | Present |
| 7 0 | a b D d | 2 | D | D H W | Present |
| 7 1 | a b d d | 2 | D | D H W | Absent |
| 7 2 | a c D D | 1 | D | D H L | Present |
| 7 3 | a c D d | 2 | D | D H L | Present |
| 7 4 | a c d d | 1 | D | D H A | Absent |
| 7 5 | b c D D | 1 | D | D W L | Present |
| 7 6 | b c D d | 2 | D | D W L | Present |
| 7 7 | b c d d | 1 | D | D W L | Absent |
| | | | 1 6 | | |

0196879

Also, the following is a method for increasing the purity of this hairless pig strain.

In accordance with Table 9, mating aadd (estimated phenotype D strain, estimated genes H, W, D) with aadd (estimated phenotype D strain, estimated genes H, W, D) gives gametes ad and bd, at F1 adad and adbd.    F2 gametes are aa, ad, da, dd, ab, ad, bd, and dd, and estimated phenotype values of the hairless pigs are H strain, HD strain 3, D strain 8; and even among D strains, because of repetitions of addd, bddd, dddd:

| 1st time | 50% | 2nd time | 75% | 3rd time | 87.5% |
| 4th time | 93.7% | 5th time | 96.9% | 6th time | 98.3% |
| 7th time | 99.2% | | | | |

Particularly in the case of 6 times or more, pigs with incorporated no-hair genes become pure hairless pigs, and as such this is an effective way of producing a pure strain of hairless pigs.

| Gene No. 81, 82 | Purity | 50% |
| Gene No. 83, 84 | Purity | 75% |
| Gene No. 85 | Purity | 100% |

## Table 9
### Method of Increasing the Purity of Hairless Breeds

0196879

| Gene No. | 7 1 | Gene No. | 7 1 |
|---|---|---|---|
| Phenotype Breed | D strain | Phenotype Breed | D strain |
| Estimated Gene | a b d d | Estimated Gene | a b d d |
| Hair Present/Absent | Absent | Hair Present/Absent | Absent |
| Gametes | a d, b d | | a d, b d |
| F 1 | a d a d, a d b d | | |
| Gametes | a a, a d, d a, d d | | a b, a d, d b, d d |

| | a a | a d | d a | d d |
|---|---|---|---|---|
| a b | a a a b | a a b d | a a b d | a b d d |
| a d | a a a d | a a d d | a a d d | a d d d |
| d b | a a b d | a b d d | a b d d | b d d d |
| d d | a a d d | a d d d | a d d d | d d d d |

| Gene No. | Estimated Gene | Number of Phenotypes | Phenotype Breed | Estimated Gene Value | Hair Present/ Absent |
|---|---|---|---|---|---|
| 7 8 | a a a b | 1 | H | HW | Absent |
| 7 9 | a a a d | 1 | H | H D | Absent |
| 8 0 | a a b d | 3 | H | HWD | Absent |
| 8 1 | a a d d | 3 | HD | H D | Absent |
| 8 2 | a b d d | 3 | DHW | DHW | Absent |
| 8 3 | a d d d | 3 | D | DH | Absent |
| 8 4 | b d d d | 1 | D | DW | Absent |
| 8 5 | d d d d | 1 | D | D | Absent |
| 1 6 | | | | | |

- Examples of uses for hairless pigs -

1. The advantage and effect of hairless pigs produced by this method is that it is easy to mutate any breed of pig to produce hairless pigs of that breed.

| | |
|---|---|
| Hairless Hampshire | Hairless Oxford Sandy Black |
| Hairless Landrace | Hairless Tamworth |
| Hairless Large White | Hairless poland china |
| Hairless Middle White | Hairless Meixan ton |
| Hairless Berkshire | Hairless Chester White |
| Hairless Duroci | Hairless Welsh |
| Hairless British Saddleback | Hairless Hereford |
| Hairless Large Black | Hairless Lacombe |
| Hairless Gloucester Old Spot | Hairless British Lop |
| Hairless Spotted | Hairless Yorkshire |

Mating these and other breeds of hairy pigs with hairless pig types provides the advantages and effect of producing hairless pig types.

2. It has the effect of reducing dust and microorganisms in the air from sticking to the pig, and prevents such dust etc. from being lifted again into the air by the movements of the pig or the air and inhaled into the pig's lungs, causing illness.

3. Eliminates external insect parasitism.

4. Facilitates disinfection and washing of the pig.

5. Stops pig hairs being mixed in with the animal's excrement, facilitating processing and treatment of the excrement into good-quality organic fertilizer and to employ biotechnology to make the excrement into food any animal feed.

The following is the result of an analysis (of March 7, 1984) of the amino acids in fecal matter. The * (1) mark indicates use of the per-formic acid method and the *(2) mark use of the barium hydroxide method. Items not marked thus were analyzed by the hydrochloric acid method.

| | |
|---|---|
| Aspartic Acid | 0.92% |
| Threonine | 0.38% |
| Serine | 0.36% |
| Glutamic Acid | 1.33% |
| Proline | 0.52% |
| Glycine | 0.56% |
| Alanine | 0.64% |

| Gystine | * (1) | 0.32% |
|---|---|---|
| Valine | | 0.50% |
| Methionine | * (1) | 0.20% |
| Isoleucine | | 0.42% |
| Leicine | | 0.76% |
| Tyrosine | | 0.27% |
| Phenylalanine | | 0.44% |
| Histidine | | 0.27% |
| Lysine | | 0.43% |
| Tryptophane | * (2) | 0.03% |
| Arginine | | 0.49% |
| Ammonia | | 0.77% |
| Protein | | 19.88% |

6. At slaughter and meat-processing time, there are no hairs in the edible meat portions where the cuts are.

7. Hairless skin can be easily processed into food.

8. Hairless ears, tails cartilage can be easily cooked and processed into food.

9. Because there are no hairs on the head, there are no hair-related problems, so the head gives a higher meat yield, which is also enhanced by the fact that all of the skin can be used for food purposes.

11. When in order to obtain specific pathogen free or aseptic pigs surgical methods in aseptic condition are employed, such as caesarean section, abdominal section, hysterectomy, hysterotomy and the like to remove the young from the mother's body, use of hairless pigs will remove the need for shaving the animals prior to such procedures. And :

1) It is easy to thoroughly attach a sealed, aseptic vinyl bag (instant isolator) around the area of incision to allow the surgical procedure to be carried out aseptically.

2) With the instant isolator in perfect contact with the skin, the young can be removed easily from the mother's body by any of the said various surgical procedures.

12. It is easy to use hairless pigs as experimental, reseach and inspection animal subjects for skin-related physicobiological experiments, research and testing of the chemistry of dyes, pigments, paints and artificial colorants.

In particular, there is no or very little cellulous antibodies. The wide range of possible applications include research and development of genetic therapy, testing of anti-carcinogenic substances in antibody production/ adjustment applications, and testing of carcinoregulatory drugs involving transplanting of human skin or cancer tissue to the hairless pigs.

13. It is easy to use hairless pigs as the animal subjects of medical experiments, research and testing.

14. The absence of hair on a hairless pig makes it easier to effectively treat burns and other injuries.

15. It is easy to utilize hairless pigs as animals for use in experiments, research, and testing in fields relating to industrial chemicals, nuclear power, nuclear fuel, radioactivity, colorants, medical treatments and polymer -related fields.

16. Pigs with no hair or with fuzz can be produced in large medium and small sizes in line with what they are intended for.

17. Large pigs being better for meat processing, large hairless pigs can be produced.

18. For use as breeding sows, medium-sized hairless pigs can be produced, as this size is advantageous for such purposes.

19. Small hairless pigs are advantageous for use in experiments, research and tests involving in fields such as medicine, pharmacology, beauty treatment, chemical production, nuclear power, nuclear fuel, radioactivity, and is other areas involving substances and organisms, and new organisms produced by genetic engineering and conversion, and radiation and the like posing a direct or indirect risk to the life of humans, animals and other living organisms.

20. Pigs with no hair or with just fuzz can be easily used as pets.

21. Carrying out the fertilization process of sperm and ova of hairless pigs outside the body and cultivating the fertilized ova to promote multiple births artificially solves the problem of immune sterility caused by the existence of antispermatozoans, while it is also possible to transplant in female pigs fertilized eggs that have been preserved by freezing, thereby developing the multiple births using frozen fertilized pig ova.

22. Pigs with no hair or just fuzz will provide a beneficial contribution to the advancement of the pig raising and food processing industries, the chemical processing industry, the medical industry, the pharmaceutical industry, and to industries utilizing nuclear power and radiation, and will also be of benefit to the life of humans, animals and other living organisms.

As described in the foregoing, hairless pigs can be reproduced by the method of the present invention, and such hairless pigs can be reproducibly developed (born) in an artificial state. In addition, hairless pigs can be expected to be more economic to feed as they should develop and fatten at a rate over and above that accounted for by the lack of hair. Also, hairless pigs produced by the method of this invention have wide utility, and if the utility is considered, the effect therefore is immeasurable.

CLAIMS:

1.       A method of producing hairless pigs comprising the steps of removing one or more fertilized ova from a sow, introducing a bacteriophage into the DNA of the hair producing chromosome in the fertilised nucleus whereby the DNA becomes a non-hair gene or inherited gene thereof, and replacing the treated ova into the or another sow.

2.       A method according to claim 1, characterised in that the bacteriophage is a corona virus of T.Epdic Gastroenteritis parasitic on Coli, and a reovirus of weak toxin rotta on Coli.

3.       A method producing hairless pigs obtained by mating pigs possessing no-hair genes  with other pigs possessing no-hair genes.

4.       A method of producing hairless pigs according to Claim 3 characterised in that the pig possessing the no-hair gene is externally hairy, said pig being obtained by mating a pig possessing the no-hair gene with a hairy pig.

5.       A method of producing hairless pigs according to claim 3, characterised in that the pig possessing the no-hair gene is obtained by mating two pigs of the same breed each of which possess the no-hair gene, said pig thus obtained being of the same type.

6.       A method of producing hairless pigs according to claim 3, characterised in that the pig possessing the no-hair gene is a hairy pig which has been given the genes of a hairless pig by genetic manipulation.

7.     A method of producing hairless pigs according to. claim 3, characterised in that a bacteriophage is introduced into the DNA of the hair-producing chromosome in the fertilized egg nucleus, the bacteriophage dissolving part of the DNA to form a no-hair gene or an inherited gene thereof.

8.     A method of producing hairless pigs according to claim 3, wherein the hairless pigs produced are characterised by not having antibodies.

FIGURE 1